# EUROPEAN PATENT APPLICATION

(11) **EP 1 962 097 A1**
(43) Date of publication of application: **27.08.2008**
(21) Application number: 07003771.8
(22) Date of filing: 23.02.2007
(51) Int. Cl.: G01N 33/82, B01D 15/36

(54) **Mass spectrometric quantitative detection of methyl malonic acid and succinic acid using hilic on a zwitterionic stationary phase**

(71) Applicant: SeQuant AB, 907 19 Umeå (SE)
(72) Inventor: Appelblad, Patrik, 90750 Umeå (SE)
(74) Representative: Linde, Jörgen

(57) **Abstract**

The invention provides a method for qualitatively and quantitatively detecting methyl malonic acid in a clinical sample that also may contain succinic acid and homocysteine, said method involving a liquid chromatography separation step followed by a mass spectroscopy detection step, said method comprising the steps of:
a) providing a sample that may contain methyl malonic acid and/or succinic acid and optionally homocysteine;
b) injecting said sample in a mobile phase comprising a high amount of water-miscible organic solvent;
c) eluting said mobile phase containing said sample through a liquid chromatography column containing zwitterionic groups covalently bound to carriers as a stationary phase;
d) detecting the possible presence of methyl malonic acid and succinic acid and optionally homocysteine by mass spectroscopy detection; and
e) determining the presence and optionally the amounts of said organic molecules using calibration data.

## Description

The present invention relates to a method for analysing small organic molecules in a large number of samples. In particular, the invention provides a method to assay the clinically interesting small organic molecules methyl malonic acid, succinic acid and optionally homocysteine in a large scale from biological matrices, such as whole blood, blood plasma, blood serum and urine.

### Technical background

All references disclosed herein are incorporated by reference.

Analytical techniques used in clinical laboratories need to be rapid, simple, and robust, but still sensitive and selective. The vast amount of samples in different matrices, (i.e. urine, plasma, serum, whole blood, etc.) requires techniques on which uncomplicated methods can be developed and used in an automatic fashion with little or no downtime. Techniques frequently in use are based on enzyme linked immunosorbent assay (ELISA) that is popular due to its ease of automatisation. However, occasionally cross-reactivity or the lack of antibodies makes it necessary to use several kinds of ELISA kits to monitor multiples of compounds that may be of clinical importance in a sample. That increases the costs and reduces the sample throughput.

Therefore liquid chromatography (LC) and particular high performance liquid chromatography (HPLC) has attracted attention as an excellent way to separate a mixture into its constituents and to determine the individual compounds quantitatively. HPLC is capable of separating either fat- or water-soluble substances into its components without any preliminary modification of such substances, by simply making a proper choice of a mobile phase and a stationary phase. However, traditional detector systems for HPLC are not sensitive to specific chemical structures but use general parameters such as absorbtion in the ultraviolet region. For example in the determination of compounds that have relatively low UV absorbtion there has been a need for pre-column derivatisation procedures to obtain retention, enhance the chromatographic separation selectivity and to achieve sufficiently high sensitivity for clinical relevant concentrations (Pastore A., et al Clin. Chem. 44, 825-32, 1998). An increasingly used tool is therefore mass spectrometric detection and US 4,298,795 discloses a HPLC system where the detection is carried out by mass spectrometry. A general introduction to mass spectrometry and in combination with liquid chromatography can be found in "Introduction to mass spectrometry" (Watson, J. Throck, Lippincott-Raven Publisher, Philadelphia, Ed. 3, 1997) and fundamentals in mass spectrometry electrospray ionisation has been described by Nadja B. Chech and Christie G. Enke in Mass Spectrometry Reviews, 20, 362-87, 2001.

Systems comprising a combination of HPLC and mass spectrometric (MS) detection have also been developed for routine analysis of a large number of clinical samples and an increased use in the clinical laboratory was recently predicted (Kinter M., Clin. Chem., 50, 1500-2, 2004). Sample preparation and calibration protocols as well as automisation routines have been optimised and adopted for HPLC-MS methods. Advances in MS technology have lead to powerful instrumentation with high resolution power within the MS instrumentation. In fact, in some cases a preceding chromatographic separation has been omitted and claimed not to be necessary.

US 6,541,263 describes determination of corticosteroids in human plasma using HPLC and mass spectroscopy. The combination of HPLC and MS renders it possible to quantitatively and qualitatively analyse low concentrations of structurally similar compounds. However, all compounds to be analysed by the technique of US 6,541,263 are fairly large and hydrophobic substances. There are also need for analysing small, polar and/or hydrophilic molecules of clinical importance.

A typical example of a clinically important analysis of such small and polar and/or hydrophilic molecules is the quantification of methylmalonic acid and succinic acid, optionally together with homocysteine. Methylmalonic acid (MMA) (as well as homocysteine) is a diagnostic marker for B 12 deficiency, while succinic acid is a physiologically abundant isomer of MMA which often interferes with the quantification of the former compound. Over the years, yet in vain, different approaches using various analytical techniques have been tested with the overall goal to allow fast and accurate quantification of MMA where the former compound is well-separated from succinic acid on a chromatographic basis, and not by deconvolution of spectral data, or using high power resolutions, i.e. MS/MS detection systems within the same run.

WO 03/079008 discloses detection of small molecules such as MMA and succinic acid using liquid chromatography and tandem MS. The method is complicated, time-consuming and involves derivatization of the sample before the assay as well as advanced calculation before obtaining a result.

WO 2006/090428 presents a reversed phase method for analyzing small molecules. It is evident from the chromatograms enclosed that it is difficult to achieve a clear separation between MMA and succinic acid.

Accordingly, there is still a need for an improved method for qualitatively and/or quantitatively analysing the presence of medically interesting small organic molecules in clinical samples. Ideally, such an assay method should be rapid, require a minimum amount of sample pre-treatment, easy to automate, and the results should be easy to construe.

### Summary of the invention

The above mentioned problems of obtaining a fast and accurate quantification of MMA are solved according to the invention by providing a method for detecting methyl malonic acid, optionally together with homocysteine and succinic acid, said method involving a liquid chromatography separation step followed by a mass spectroscopy detection step, said method comprising the steps of:
a) providing a sample that may contain methyl malonic acid and/or succinic acid and optionally homocysteine;
b) injecting said sample in a mobile phase comprising a high amount of water-miscible organic solvent;
c) eluting said mobile phase containing said sample through a liquid chromatography column containing zwitterionic groups covalently bound to carriers as a stationary phase;
d) detecting the possible presence of methyl malonic acid and/or succinic acid and optionally homocysteine by mass spectroscopy detection; and
e) determining the presence and optionally the amounts of said organic molecules using calibration data.

Since the sample pre-treatment protocol is known by dilution factors the original sample concentration can be calculated and related to other observations of the object (patient) in study for clinical or medicinal evaluation.

Preferably, the water-miscible organic solvent is acetonitrile. It may also be selected from a group of water soluble solvents consisting of methanol, ethanol, propanol, acetone, and THF or mixtures thereof. Acetonitrile is preferred.

Furthermore, it is preferred that the mobile phase is constituted of a mixture of acetonitrile and an aqueous buffer solution, such as an aqueous solution of a salt, a weak acid and a weak base. The buffering substance is preferably a salt such as ammonium acetate but ammonium formiate, and weak acids such as formic acid, or acetic acid may also be used. In some HILIC/MS applications it is possible to include ammonia and ammonium carbonate in the aqueous buffer solution. The aqueous buffer solution may have a slightly acidic to slightly basic pH, preferably within the range of 4.0 - 7.5, preferably within the range of 4.0 - 7.0, and in particular within the range of 4.0 - 5.0, and the overall ionic strength of the mobile phase is preferably within the range of 5 - 60 mM.

Furthermore, it is preferred that the mobile phase has a composition of acetonitrile/aqueous buffer solution within the range of 95/5 - 60/40, preferably 85/15 - 65/35 and most preferably 80/20 - 65/35.

Furthermore, it is also preferred that the zwitterionic groups of the stationary phase are CH₂-N⁺(CH₃)₂-CH₂-CH₂-SO₃⁻ or -O-PO⁻₃-CH₂-CH₂-N⁺(CH₂)₃. Stationary phases that can be used when carrying out the present invention are disclosed in US 2005/0064192 A1 and WO 00/27496.

The selectivity and suitability for separation of polar and hydrophilic compounds of these zwitterionic stationary phases have proved to be rather independant of stationary phase carrier chemistry or physical format. For example it was shown using a zwitterionic stationary phase on a silica carrier that changes in mobile phase pH could be used to optimise separation selectivity for peptides (P. J. Boersema, N. Divecha, A. J. R. Heck, S. Mohammed J. Proteome Res., (2007) in press) and a similar pattern was seen for a series of benzoic acids on a polymeric methacrylate based zwitterionic monolith (Z. Jiang, N.W. Smith, P.D. Ferguson, and M.R. Taylor, Anal. Chem.79,1243-1250, 2007). No effect was attributed to the carrier material in these studies, but to dissociation and corresponding change in hydrophilicity of the analytes influencing both the capacity factors and selectivity. The impact of stationary phase functional groups and carriers was discussed in a recent review on HILIC (P. Hemström, K. Irgum J. Sep. Sci., 29, 1784-1821, 2006)

Examples of carriers that are suitable in connection with the present invention are porous silica, zirconium, graphite, and polymer or copolymer materials in the shape of spherical particles having a size ranging from 1 to 30 µm, and preferably within the range of 3.5 - 10µm, and a porosity from 50 to 300 Å, or a monolithic structure of said materials, or alternatively the inner-wall of narrow bore capillaries.

The polymer or copolymer carrier of synthetic or natural origin may comprise mono-or oligovinyl monomer units such as styrene and its substituted derivatives, acrylic acid or methacrylic acid, alkyl acrylates and methacrylates, hydroxyalkyl acrylates and methacrylates, acrylamides and methacrylamides, vinylpyridine and its substituted derivatives, divinylbenzene, divinylpyridine, alkylene diacrylate, alkylene dimethacrylate, oligoethylene glycol diacrylate and oligoethylene glycol dimethacrylate with up to five ethylene glycol repeat units, alkylene bis(acrylamides), piperidine bis(acrylamide), trimethylolpropane triacrylate, trimethylolpropane trimethacrylate, pentaerythriol triacrylate and tetraacrylate, and mixture thereof.

In a preferred embodiment, the zwitterionic functional ligand has been bound to said carrier by graft polymerization or by a multi-step reaction attachment of zwitterionic monomers or zwitterionic ligands onto the surface of said carrier or by a copolymerisation of a said monomers or ligands and a cross-linker monomer and diluents forming a monolith carrier containing the zwitterionic functional groups.

It is especially advantageous to apply the method for quantitatively or qualitatively determining methylmalonic acid, optionally together with homocysteine and succinic acid, in clinical samples. Such clinical samples are preferably body fluids, such as urine, blood, blood plasma, and blood serum. The clinical samples are mixed with acetonitrile resulting in precipitation of proteins that could be contained in said samples. After removing precipitated proteins the remaining supernatant could be directly injected into the mobile phase and eluated.

### Description of the figures

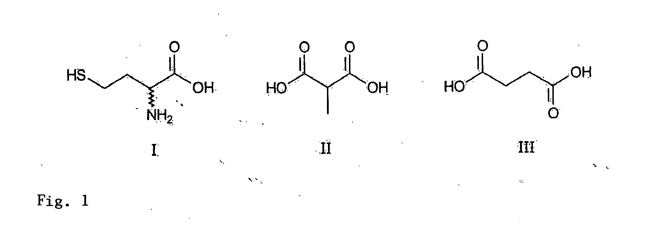

The present invention will be further described with reference to the enclosed figures, in which
Figure 1 discloses the structure of MMA, Succinic Acid and Homocysteine.
Figure 2 discloses the zwitterionic betain functional group of the ZIC-HILIC column.
Figure 3 a-c presents chromatogram illustrating the separation of MMA in presence of deuterated MMA internal standard from human plasma samples spiked with a) 50, b) 476 and c) 1000 nM MMA, respectivelyusing HILIC in combination with single ion monitoring negative electrospray MS.
Figure 4 presents a calibration curve on MMA in human plasma samples spiked with 50 to 1000 nM at six of each other independent concentration levels.
Figure 5 presents a calibration curve on MMA in human plasma samples spiked with 50 to 200 000 nM at 13 of each other independent concentration levels; and
Figure 6 shows chromatograms of full baseline HILIC separation of homocysteine, methylmalonic acid and succinic acid, followed by detection by UV (a) and MS (b), respectively. Experimental conditions used at these experiments differs from those used for optimal MMA quantitation, and exemplifies the possibility of achieving quantitative or qualitative determination of methyl malonic acid, together with homocysteine and succinic acid in clinical samples.

### Detailed description of the invention

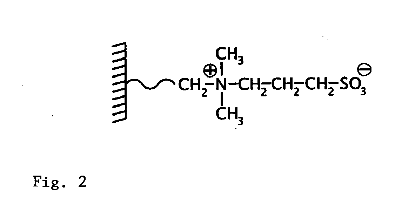

The invention provides a feasible alternative to reversed phase HPLC methods and the well-known combination of standard-HPLC and mass spectroscopy, namely hydrophilic interaction liquid chromatography (HILIC) combined with mass spectrometric detection. HILIC is a separation technique suitable for polar and hydrophilic compounds, and employs an eluent containing a high content (40 to 99% v/v) of water miscible organic solvent (acetonitrile, propanol, ethanol, methanol, THF, acetone) to promote hydrophilic interactions between the analyte and a hydrophilic stationary phase. It is generally found that the mobile phases used in HILIC are well suited for ESI-MS detection (Guo Y., Gaiki S., J. Chromatogr. A, 1074, 71-80, 2005) and often enhances the detection limits compared to reversed phase methods, in particular, while analyzing hydrophilic compounds and when the final mobile buffer salt concentration can be kept at reasonable concentration levels, typically below 50 mM (Bengtsson J., Jansson B., Hammarlund-Udenaes M., Rapid Commun. Mass Spectrom., vol. 19 (2005), 2116 - 2122). Although there are other HILIC stationary phases commercially available, none is truly comparable with the stationary phases disclosed in WO, A1, 00/27496 and US 2005/0064192 A1. Such stationary phases are sold under the registered trade marks ZIC®-HILIC and ZIC®-pHILIC phases (SeQuant AB, Sweden). Columns containing these highly polar zwitterionic stationary phases provide a unique environment particularly capable of solvating polar and charged compounds, which enables high performance HILIC separations. The zwitterionic stationary phase, Figure 2, can interact with charged analytes via weak electrostatic interactions, and in practice, this provides the chromatographer with a larger degree of freedom when choosing among buffer salts and ionic strength in method development, thus making the column an ideal choice for LC-MS analysis. This particular patent application note illustrates advantages when combining efficient separation with a sensitive detection principle, and is exemplified by an isocratic HILIC separation capable of resolving methylmalonic acid from succinic acid and homocysteine in clinical samples.

The ZIC®-HILIC columns (SeQuant AB, Sweden) are silica based stationary phases with either 3.5, 5 or 10 µm particle size, while the ZIC®-pHILIC columns (SeQuant AB, Sweden) are polymer-based stationary phases with 5 µm particle size, yet both having a sulfobetaine type zwitterionic functionality.

### Example 1

### Experimental section

**Reagents and Chemicals.** Acetonitrile (HPLC grade), and the ammonium acetate of analytical grade were both purchased from form Merck (Darmstadt, Germany), while formic acid (%, p.a.) was from JT BAKER (Deventer, The Netherlands). All water was purified by a Milli-Q water purification system (Millipore, Bedford, MA). The plasma protein precipitation (PPT) solution was prepared by adding 25 mL acetonitrile, followed 250 microliter concentrated acetic acid and 43 microliter of a 196.5 micromolar deuterated methyl malonic acid (d3-MMA) stock solution to a 50 mL volumetric flask to which more acetonitrile was added upto the mark. This PPT solution contains 99.5 volume-% acetontrile, 0.5 volume-% acetic acid and a 169 nM concentration of d3-MMA.

### Protein Precipitation and Clean-up of Plasma Samples.

Using the HILIC technique, it is possible to apply a very effective and straight forward sample pre-treatment of clinical samples. Since acetonitrile is the weak solvent in HILIC, it is possible to precipitate interfering plasmaproteins in acetonitrile. PPT solution (0.80 mL) was first pipetted into a 2 mL glass autosampler vial whereafter human plasma (0.20 mL) was added and the vials were mounted with inert vial caps. All samples were then allowed to stand on an orbital shaker (Janke&Kunkel, Typ Vx8) (or other equipment with similar performance) for five minutes to promote adequate mixing. Following the plasma protein precipitation process, the vials were transferred into a Hettich Zentrifugen Rotana 460R centrifuge (or other equipment with similar performance) operating at 6200 rpm for ten minutes at 15 degrees Celcius prior to analysis. To determine analytical recovery, known amounts of MMA and MMA-d3 were added before and after the protein precipitation.

**Chromatographic System**. The chromatographic system consisted of an Agilent 1100 separation module which delivered a mobile phase containing acetonitrile and a 100 mM aqueous ammonium acetate buffer (80:20 v/v), pH 4.7 at a flow rate of 0.4 mL/min. Standards, spiked samples, controls, and human patient samples (4 µL) were injected using an Agilent 1100 autosampler, onto a 100 mm long by 2.1 mm i.d. ZIC®-HILIC separation column having 3.5 micron particles that were placed in an Agilent 1100 column oven set at 30°C. Quantitation were carried out using single ion monitoring (m/z 117.2 and 120.2) in negative mode ESI on an Agilent 1100 Mass Spectrometer and applying a drying gasflow set at 10L/min, gas temperature: 300 °C, and the capillary voltage set at 3.0 KV. All chromatograms were recorded on an Agilent Chemstation.

### Quantitation of Methyl Malonic Acid (MMA) in Human plasma samples using negative ESI MS detection after separation in HILIC mode on a Zwitterionic

**Stationary Phase.** Work have been carried out at obtaining analytical data for repeatability, reproducibility, precision, linearity, LOD etc for MMA, using standard solutions, spiked plasma samples, as well as human patient samples, in total presented elsewhere. A simple but yet rugged and efficient procedure for sample work-up followed by negative ESI- MS single ion monitoring for quantitation have been developed for biological samples using isotopically labelled internal standards. Excellent sensitivity and adequate linearity (r² > 0,998) are achieved with the current system set-up, and where LOQ for MMA is approximately 10 nM and LOD less than 5 nM for standard solutions. As the endogeneous levels for MMA are ranging between 140-500 nM, and our proposed sample procedure via dilution/protein precipitation will dilute samples four times, target levels are reached.

### Results:

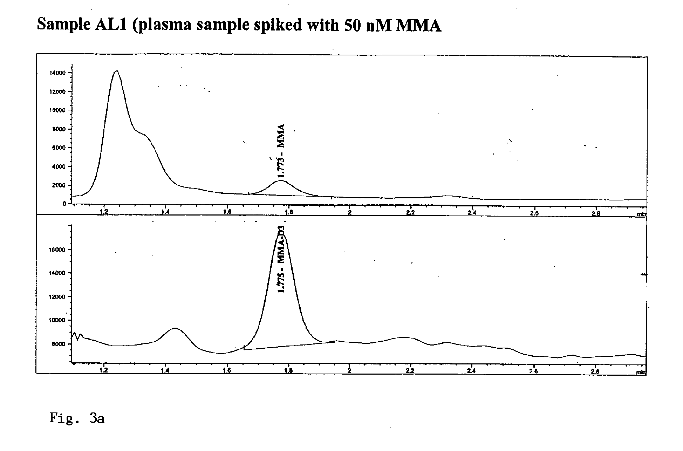

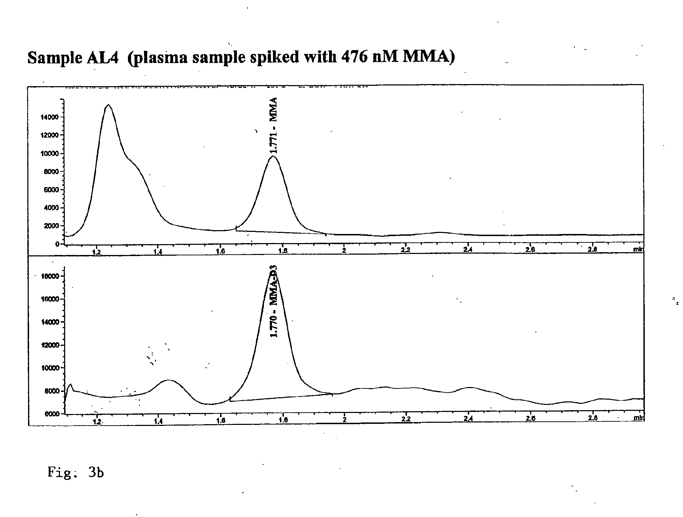

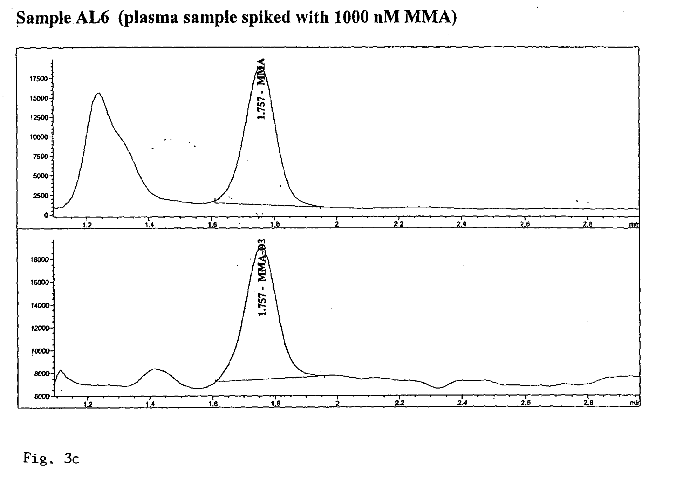

Figure 3 a-c presents chromatogram illustrating the separation of MMA from human plasma samples spiked with a) 50, b) 476 and c) 1000 nM MMA, respectively. To all samples were deuterated labeled internal standard (MMA-d3) added, see bottom trace chromatograms in Figure 3a-c. All separations were carried out on a 100x2.1 mm ZIC-HILIC column having 3.5 micron particles, and detection carried out using single ion monitoring (m/z 117.2 and 120.2) in negative mode ESI on an Agilent 1100 Mass Spectrometer.

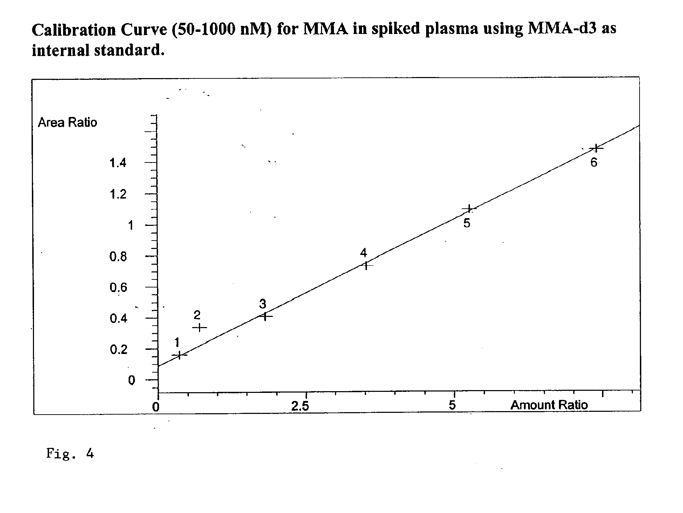

Figure 4 presents a calibration curve on MMA in human plasma samples spiked with 50 to 1000 nM at six of each other independent concentration levels. To all samples were constant amount of d internal standard (MMA-d3) added and the area response ratio between MMA and MMA-d3 were plotted.

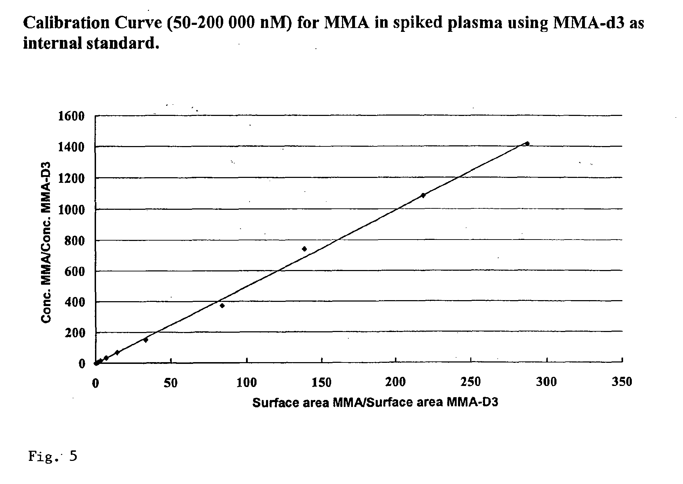

Figure 5 presents a calibration curve on MMA in human plasma samples spiked with 50 to 200 000 nM at 13 of each other independent concentration levels. To all samples were constant amount of d internal standard (MMA-d3) added and the area response ratio between MMA and MMA-d3 were plotted.

### Example 2

| | |
|---|---|
| **Experimental conditions** Column: | ZIC®-HILIC 50 x 4.6 mm, 5 µm |
| **UV** | |
| Column temp: | RT |
| Mobile phase: | Acetonitrile/ammonium acetate (pH 6.8, 30 mM in final solution); 70/30 (v/v) |
| Flow-rate: | 1.5 mL/min |
| Detector: | UV at 206 nm (UFS 1.0 V) |
| Injection volume: | 5µL of test solution in mobile phase |
| **MS** | |
| Column temp: | 30°C |
| Mobile phase: | Acetonitrile/ammonium acetate (pH 6.8, 25 mM in final solution); 75/25 (v/v) |
| Flow-rate: | 1.0 mL/min |
| Split: | 100 µL/min to MS |
| Detector: | Agilent 1100 bench top MS, ESI in positive mode |
| Capillary voltage: | 3000 V |
| Fragmentor: | 150 V |
| Mass range: | 50-200 m/z |
| Injection volume: | 5 µL of 0.1 mg/mL of each compound in mobile phase |
| Sample: | In elution order; homocysteine, methylmalonic acid and succinic acid all dissolved in mobile phase. |

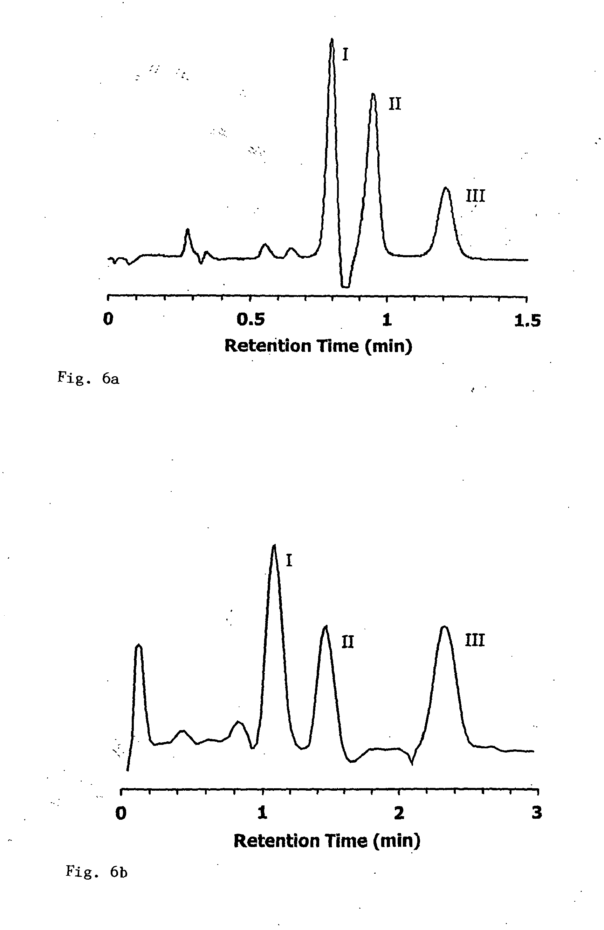

Method development is commonly performed using UV detection, because of its ease of use and robustness, but the technique often lacks the sensitivity needed to allow quantification at relevant physiological levels. Herein, it is illustrated that provisional optimal separation conditions can be established via UV-detection, Figure 6(a), and then easily transferred and slightly modified to better fit MS detection in order to gain sensitivity. Baseline separation for all compounds can be achieved within 90 seconds, and that the compounds elute with a k' between 1.4 and 3. Worth noting is the dip in between homocysteine and methylmalonic acid. The rationale for the phenomena is a combination of low detection wavelength and a slight mismatch in buffer concentration between the sample and the mobile phase. By lowering the flow-rate and the aqueous portion in the mobile phase (from 30 to 25 % volume), and slightly decreasing the ionic strength the method was adjusted to the LC-MS instrument and sufficient separation efficiency was reached, as seen in Figure 6(b). Using provisional MS compatible conditions, determination of methyl malonic acid, together with homocysteine and succinic acid is possible in biological samples but not at clinical relevant concentration levels. Figure 6 thus illustrates separation of homocysteine, methyl malonic acid and succinic acid followed by detection by UV (a) and MS (b), respectively. However, the experimental conditions used at all these experiments differ from those used for optimal MMA quantitation.

The ZIC®-HILIC column is indeed a suitable tool for separation of methylmalonic acid and succinic acid. Combined with relatively inexpensive and uncomplicated MS detection equipment, physiological relevant concentration can easily be quantified with the possibility of processing up to 20 samples per hour.

## Claims

1. A method for qualitatively and/or quantitatively detecting methyl malonic acid in a clinical sample that also may contain succinic acid and/or homocysteine, said method involving a liquid chromatography separation step followed by a mass spectroscopy detection step, said method comprising the steps of:
a) providing a sample that may contain methyl malonic acid and/or succinic acid and optionally homocysteine;
b) injecting said sample in a mobile phase comprising a high amount of water-miscible organic solvent;
c) eluting said mobile phase containing said sample through a liquid chromatography column containing zwitterionic groups covalently bound to carriers as a stationary phase;
d) detecting the possible presence of methyl malonic acid and succinic acid and optionally homocysteine in said sample by mass spectroscopy detection; and
e) determining the presence and optionally the amounts of said organic molecules using calibration data.

2. A method according to claim 1, **characterised in that** the water-miscible organic solvent is acetonitrile or optionally selected from a group of water soluble solvents consisting of methanol, ethanol, propanol, acetone, and THF or mixtures thereof.

3. A method according to claim 2, **characterised in that** the mobile phase is a mixture of acetonitrile and an aqueous buffer solution, said aqueous buffer solution having a slightly acidic to slightly basic pH, preferably within the range of 4.0 - 7.5, more preferably within the range of 4.0 - 7.0 and in particular within the range of 4.0 - 5.0, and where the overall ionic strength of said mobile phase is within the range of 5 - 60 mM.

4. A method according to claim 3, **characterised in that** said aqueous buffer solution is an aqueous solution of a substance chosen from the group of ammonium acetate, ammonium formiate, formic acid, acetic acid, ammonia, and ammonium carbonate.

5. A method according to claim 3 or claim 4, **characterised in that** the mobile phase has a composition of acetonitrile/aqueous salt solution within the range of 95/5 - 60/40, preferably 85/15 - 65/35, and most preferably 80/20 - 65/35.

6. A method according to claim 1, **characterised in that** the zwitterionic groups of the stationary phase are CH₂-N⁺(CH₃)₂-CH₂-CH₂-SO₃⁻ or -O-PO⁻₃-CH₂-CH₂ -N⁺(CH₂)₃, preferably CH₂-N⁺(CH₃)₂-CH₂-CH₂-SO₃⁻.

7. A method according to claim 6, **characterised in that** said zwitterionic groups are bound to silica particles or polymer particles.

8. A method according to claim 7, **characterised in that** said particles have a particle size within the range of 1 - 30 µm, preferably within the range of 3.5 - 10 µm.

9. A method according to claim 1, **characterised in that** said sample is a clinical sample, such as blood plasma, whole blood, blood serum and urine and that said sample is mixed with acetonitrile thereby precipitating proteins in said sample, said precipitated proteins being removed from said sample, said sample being directly injected into the mobile phase without any further pre-treatment.
